(19) Europäisches Patentamt
European Patent Office
Office européen des brevets

(11) Veröffentlichungsnummer: **0 015 516**
**B1**

# (12) EUROPÄISCHE PATENTSCHRIFT

(45) Veröffentlichungstag der Patentschrift: **19.05.82**

(51) Int. Cl.³: **C 07 D233/64**

(21) Anmeldenummer: **80101008.3**

(22) Anmeldetag: **29.02.80**

## (54) Verfahren zur Herstellung von 5-Hydroxymethylimidazolen.

(30) Priorität: **02.03.79 DE 2908212**

(43) Veröffentlichungstag der Anmeldung: **17.09.80 (Patentblatt 80/19)**

(45) Bekanntmachung des Hinweises auf die Patenterteilung: **19.05.82 Patentblatt 82/20**

(84) Benannte Vertragsstaaten: **CH DE FR GB IT**

(56) Entgegenhaltungen:
**EP - A - 0 003 052**
**EP - A - 0 004 534**
**DE - A - 2 659 851**

**Chemical Abstracts Band 82, Nr. 7, 17. Februar 1975 Columbus, Ohio, USA M. MASUI et al. « Reaction of 2,4(5)-dialkylimidazoles with formaldehyde » Seite 435, Spalte 1, Abstract Nr. 43347 d**

**Chemical Abstracts Band 78, Nr. 8, 26. Februar 1973 Columbus, Ohio, USA E.F. GODEFROI et al. « Synthesis of 1,2-disubstituted imidazole-5-carboxalydehydes and -4,5-dicarboxaldehydes » Seite 512, Spalte 2 bis Seite 513, Spalte 1, Abstract Nr. 58317 k**

(73) Patentinhaber: **BASF Aktiengesellschaft**
**Carl-Bosch-Strasse 38**
**D-6700 Ludwigshafen (DE)**

(72) Erfinder: **Kempe, Uwe, Dr.**
**Carl-Bosch-Strasse 44**
**D-6703 Limburgerhof (DE)**
Erfinder: **Dockner, Toni, Dr.**
**Grossgasse 6**
**D-6701 Meckenheim (DE)**
Erfinder: **Frank, Anton**
**Bannwasserstrasse 72**
**D-6700 Ludwigshafen (DE)**
Erfinder: **Karn, Helmut**
**Sternstrasse 120**
**D-6700 Ludwigshafen (DE)**

**EP 0 015 516 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

# Verfahren zur Herstellung von 5-Hydroxymethylimidazolen

Die Erfindung betrifft ein Verfahren zur Herstellung von 5-Hydroxymethylimidazolen in Form ihrer Hydrochloride durch Hydroxymethylierung in wäßriger salzsaurer Lösung.

Die Chlormethylierung aromatischer Verbindungen durch Umsetzung mit Formaldehyd in Gegenwart von konzentrierter Salzsäure und gegebenenfalls in Gegenwart eines Katalysators, wie Zinkchlorid oder Aluminiumchlorid, ist eine wohlbekannte Reaktion. Ebenso ist die basenkatalysierte Kondensation reaktionsfähiger Aromaten, z.B. Phenol, Pyrrol, Indol, Chinolin und deren Derivaten mit Formaldehyd zu Hydroxymethylverbindungen in der Literatur gut beschrieben (J. Mathieu u. J. Weill-Raynal, Formation of C-C-Bonds, Seiten 4 bis 10 und 29 bis 31, Georg-Thieme-Verlag (1973)). Die Hydroxymethylierung von Aromaten, speziell von Imidazol, unter salzsauren Bedingungen ist dagegen nicht bekannt.

So wird beispielsweise die Herstellung von 4-substituierten 5-Hydroxymethylimidazolen nur am Beispiel des 4-Methyl-5-hydroxymethylimidazols durch Umsetzung von 4-Methylimidazol mit Formaldehyd beschrieben (J. Chem. Soc. 99, Seite 2055 (1911)).

Nachteilig an diesem Verfahren sind die niedrigen Ausbeuten, die geringe Selektivität und die Aufarbeitung des Rohprodukts mit Pikrinsäure, so daß nur schwer eine reine 5-Hydroxymethylverbindung erhalten werden kann. Wegen der Schwierigkeit, diese Verbindungen auf diesem Wege herzustellen, wird 4-Methyl-5-hydroxymethylimidazol gemäß der DE-OS 26 37 670 aus 4-Methyl-5-carbonsäureestern durch Reduktion mit Alkalimetallen oder Calcium in flüssigem Ammoniak hergestellt. Dieses Verfahren ist langwierig und im technischen Maßstab wegen der Anwendung von flüssigem Ammoniak und Alkalimetallen schwierig durchzuführen. Ebenso umständlich und noch teurer ist die Herstellung von 4-Methyl-5-hydroxymethylimidazol durch Reduktion eines entsprechenden Carbonsäureesters mit Lithiumaluminiumhydrid (J. Med. Chem. *19*, Seiten 923 bis 928, 1976).

In der DE-OS 28 00 148 wird die Herstellung von 4-Methyl-5-hydroxymethylimidazol in Form des Hydrochlorids durch Hydrolyse von 4-Methyl-5-chlormethylimidazol vorgeschlagen, indem man 4-Methyl-5-chlormethylimidazol-hydrochlorid in wäßriger Lösung auf 50 bis 60 °C erwärmt. In einem vorherigen separaten Reaktionsschritt wird das 4-Methyl-5-chlormethylimidazol-hydrochlorid durch Erhitzen von Formaldehyd und 4-Methylimidazol in konzentrierter wäßriger salzsaurer Lösung hergestellt.

Weiterhin wird in der DE-OS 28 25 547 die Herstellung von 5-Hydroxymethylimidazolen aus den entsprechenden 1-Hydroxymethylimidazolen durch Umlagerung in wäßriger Salzsäure vorgeschlagen. Die 1-Hydroxymethylimidazole selbst werden für sich durch Umsetzung des Imidazols mit Paraformaldehyd oder Trioxan zweckmäßig in Gegenwart eines aromatischen Kohlenwasserstoffes, insbesondere Toluol, hergestellt.

« In Chem. Pharm. Bull., Band 22, Nr. 10, 1974, Seiten 2359 bis 2364, wird die Hydroxymethylierung von 2,4-Dialkylimidazolen in einem schwach sauren, abgepufferten Medium beschrieben. Dabei werden mäßige Ausbeuten zwischen 10,6 und 26,5 % erhalten und in etwa gleicher Menge zwischen 8,1 und 26,0 % ein Dihydropyrazinderivat als Nebenprodukt. Gemäß den Angaben auf Seite 2362 kann die Umsetzung mit Formaldehyd nur dann verlaufen, wenn der Imidazolring unter den Reaktionsbedingungen nicht vollständig protoniert vorliegt. »

Aufgabe der vorliegenden Erfindung ist es, 5-Hydroxymethylimidazole in möglichst einfacher Weise und frei von Nebenprodukten in technischem Maßstabe herzustellen.

Überraschenderweise wurde nun gefunden, daß man 5-Hydroxymethylimidazole der Formel 1

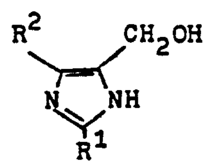

in der $R^1$ Wasserstoff, einen Alkylrest mit 1 bis 18 C-Atomen, Phenyl oder Aralkyl und $R^2$ einen Alkylrest mit 1 bis 18 C-Atomen oder Phenyl bedeuten, durch Hydroxymethylierung mit Formaldehyd direkt in einstufiger Reaktion herstellen kann, durch Umsetzung eines Imidazols der Formel 2

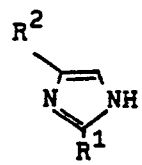

in der $R^1$ und $R^2$ die für Formel 1 angegebenen Bedeutungen haben, mit Formaldehyd oder einem Oligomeren des Formaldehyds in wäßriger salzsaurer Lösung mit einem Gehalt von 5 bis 18 Gew.-% Chlorwasserstoff, bezogen auf das vorhandene Wasser, bei Temperaturen von 80 bis 160 °C und gegebenenfalls in einem geschlossenen System unter Druck bei einem Molverhältnis von Imidazol zu Chlorwasserstoff von 1 : 1,5 bis 1 : 5 und anschließender Isolierung des erhaltenen 5-Hydroxymethylimidazols in Form seines Hydrochlorids.

Als Aralkyl sind insbesondere Benzyl, β-Phenylethyl sowie die höheren Homologen zu verstehen.

Bevorzugt werden nach dem erfindungsgemäßen Verfahren 5-Hydroxymethylimidazole hergestellt, in denen $R^1$ Wasserstoff oder einen Alkylrest mit 1 bis 4 C-Atomen und $R^2$ einen Alkylrest mit 1 bis 4 C-Atomen bedeuten.

Das erfindungsgemäße Verfahren wird bevorzugt bei Temperaturen von 100 bis 140 °C durchgeführt. Gemäß den angewandten Temperaturen stellt sich, wenn man in einem geschlossenen System arbeitet, beispielsweise in Emailkesseln oder korrosionsstabilen Metallautoklaven, der entsprechende Druck ein.

Der Formaldehyd wird in üblicher wäßriger Lösung, enthaltend 15 bis 40 Gew.-% Formaldehyd, gasförmig oder in Form seiner Oligomeren, wie Paraformaldehyd oder 1,3,5-Trioxan, verwendet. Das Molverhältnis Imidazol zu Formaldehyd liegt vorteilhaft bei 1 : 1 bis 1 : 1,5.

Innerhalb der angegebenen Konzentration von 5 bis 18 Gew.-% Chlorwasserstoff ist ein Gehalt von 12 bis 16 Gew.-% Chlorwasserstoff bevorzugt.

Die Umsetzung ist in der Regel innerhalb eines Zeitraumes von 20 bis 60 Stunden beendet.

Nach Beendigung der Reaktion kann in an sich üblicher Weise aufgearbeitet werden, indem man die wäßrig salzsaure Lösung bei Temperaturen von 10 bis 120 °C, bevorzugt 30 bis 45 °C, eingengt und das erhaltene Hydrochlorid nach dem Waschen mit Aceton oder Methanol oder einem anderen niederen einwertigen Alkohol mit 2 bis 6 C-Atomen durch Abfiltrieren isoliert und anschließend trocknet.

Ein ganz besonders reines 5-Hydroxymethylimidazol wird erhalten, indem man das Reaktionsgemisch mit Alkali, bevorzugt 20 bis 60 gew.-%iger Natronlauge, auf einem pH-Wert von 7 bis 12 stellt und mit einem mit Wasser nicht mischbaren organischen Extraktionsmittel, zweckmäßigerweise Butanol, Pentanol oder 2-Äthylhexanol, extrahiert und anschließend durch Einleiten von Chlorwasserstoff das Hydrochlorid wieder ausfällt. Mit dieser bevorzugten Arbeitsweise ist es möglich, technisches 4-Methylimidazol mit einer Reinheit von 85 bis 93 % zu verwenden, da Nebenprodukte des Ausgangsmaterials wegen der geringen Löslichkeitsunterschiede der Hydrochloride in Wasser nur unter großem Aufwand vom 4-Methyl-5-hydroxymethylimidazol abzutrennen sind. Trotz der extrem guten Wasserlöslichkeit der erhaltenen 5-Hydroxymethylimidazole gelingt die Abtrennung mit den genannten Lösungsmitteln ohne Schwierigkeiten.

Vorzugsweise werden etwa 50 bis 80 % des Extraktionsmittels vor dem Ausfällen bei einer Temperatur von 5 bis 100 °C, speziell aber bei 10 bis 50 °C, abdestilliert; von etwas anfallendem Kochsalz wird abfiltriert und bei 5 bis 45 °C gasförmiger Chlorwasserstoff eingeleitet. Gegebenenfalls kann aus einem niederen einwertigen Alkohol mit 1 bis 6 C-Atomen, beispielsweise Methanol oder iso-Propanol, der bis zu 25 % (v/v) Wasser enthalten kann, umkristallisiert werden.

Gegenüber dem oben genannten zweistufigen Verfahren zeichnet sich das erfindungsgemäße Verfahren dadurch aus, daß es ohne organische Lösungsmittel in einer einstufigen Reaktion ausschließlich und in hohen Ausbeuten zu dem gewünschten 5-Hydroxymethylimidazol führt.

Es wird darauf hingewiesen, daß der Fachmann erwarten würde, daß bei den für die Reaktion erforderlichen Temperaturen und Reaktionszeiten eine Gleichgewichtsverschiebung zu den 5-Chlormethylverbindungen eintreten könnte. Interessanterweise verläuft die Hydroxymethylierung unter den erfindungsgemäßen Bedingungen in salzsauren wäßrigen Lösungen innerhalb des Konzentrationsbereiches von 5 bis 18 Gew.-% Chlorwasserstoff, bezogen auf das Wasser, ohne die Bildung von 5-Chlormethylverbindungen. Daß ausschließlich reines 5-Hydroxymethylimidazol ohne Nebenreaktionen und ohne Verunreinigungen durch Chlormethylverbindungen gebildet wird, war nicht zu erwarten.

Auf gleiche Weise werden beispielsweise neben den in den Beispielen genannten Verbindungen folgende 5-Hydroxymethylimidazole hergestellt:

2-Phenyl-4-methyl-5-hydroxymethylimidazol
2-Isopropyl-4-methyl-5-hydroxymethylimidazol
2-Methyl-4-ethyl-5-hydroxymethylimidazol
2-Methyl-4-phenyl-5-hydroxymethylimidazol
2-Benzyl-4-methyl-5-hydroxymethylimidazol.

Das erfindungsgemäße Verfahren soll durch folgende Beispiele beschrieben, aber nicht eingeschränkt werden. Gewichtsteile verhalten sich zu Volumenteilen wie das Kilogramm zum Liter.

Beispiel 1

82 Gew.-Teile 4-Methylimidazol (Reinheit 98,7 %), 560 Vol.-Teile Wasser, 450 Gew.-Teile wäßrige Salzsäure (ca. 37 Gew.-%) und 100 Gew.-Teile einer wäßrigen Formaldehydlösung (30 Gew.-%) werden unter Rückfluß in einem geschlossenen Gefäß 48 Stunden bei 120 °C erhitzt. Nach dem Abdestillieren des Wassers erhält man 160 Gew.-Teile Rückstand. Dieser wird aus 400 Vol.-Teilen Ethanol umkristallisiert. Nach dem Trocknen im Vakuum erhält man 91 Gew.-Teile 4-Methyl-5-hydroxymethylimidazolhydrochlorid vom Schmelzpunkt 225,2 bis 232,3 °C. Aus der Mutterlauge werden nach dem Einengen weitere 21 Gew.-Teile und aus dem Rückstand der Mutterlauge nochmals 5 Gew.-Teile der gleichen Verbindung erhalten.

Die Rohausbeute beträgt 117 Gew.-Teile (78,8 % d.Th.).

Beispiel 2

Die Umsetzung wird gemäß Beispiel 1 durchgeführt, wobei Gewichtsteile Gramm und Volumenteile Milliliter bedeuten, jedoch wird die wäßrige salzsaure Reaktionslösung nach 48 Stunden Reaktionszeit bei 120 °C mit Eis auf 20 bis 30 °C abgekühlt und bei dieser Temperatur mit Natronlauge (60 Gew.-%) ein pH von 8,5 eingestellt. Diese Lösung wird dreimal mit je 1 l Butanol ausgeschüttelt und die vereinigte butanolische Phase auf ein Volumen von ca. 1 l eingeengt. Nach dem Abkühlen wird ein geringer Niederschlag an Kochsalz abfiltriert und in die Lösung werden unter Eiskühlung bei einer

Temperatur von 20 bis 30 °C 40 g Chlorwasserstoff eingeleitet. Die Lösung wird auf 10 °C abgekühlt und 2 Stunden bei dieser Temperatur stehengelassen. Man erhält 83 g 4-Methyl-5-hydroxymethylimidazol-hydrochlorid vom Schmelzpunkt 222-231. Nach dem Einengen des Filtrats bis zur Kristallisation werden nochmals 17 g vom Schmelzpunkt 206-229,5 °C erhalten. Der Rückstand aus der Mutterlauge beträgt 30,5 g ; durch Anrühren mit 30 ml i-Propanol können weitere 7 g vom Schmelzpunkt 192,3-207,1 °C erhalten werden. Die Proben sind nach dem NMR-Spektrum und High-Pressure-Liquid-Chromatographie identisch. Die Rohausbeute beträgt daher 107 g (72,1 % d.Th.). 83 g dieses Produkts werden mit 210 ml wäßrigem n-Propanol umkristallisiert. Man erhält 60 g 4-Methyl-5-hydroxymethylimidazol-hydrochlorid vom Schmelzpunkt 230,5-239 °C.

Beispiel 3

Ansatz wie 2, jedoch wird dreimal mit je 1 l Ethylhexanol ausgeschüttelt. Nach dem Einengen und Ausfällen gemäß Beispiel 2 werden 60,5 g (40,7 % d.Th.) 4-Methyl-5-hydroxymethylimidazol-hydrochlorid vom Schmelzpunkt 206,7-214,5 °C erhalten.

Beispiel 4

Ansatz wie 2, jedoch wird sechsmal mit je 250 ml Pentanol ausgeschüttelt. Nach dem Einengen der Pentanol-Phase auf 500 ml wird gemäß Beispiel 1 verfahren. Die Rohausbeute beträgt 79,5 g (53,5 % d.Th.) vom Schmelzpunkt 206,5-214 °C.

Beispiel 5

82 Gew.-Teile 4-Methylimidazol (99 %ig) werden in eine Lösung aus 245 Vol. Teilen Wasser und 225 Gew.-Teilen konzentrierter wäßriger Salzsäure (ca. 36 Gew.-%) eingetragen und mit 105 Gew.-Teilen wäßriger Formaldehydlösung (ca. 30 Gew.-%) 24 Stunden bei 130 °C und dem sich einstellenden Druck unter Rückfluß erhitzt.

Die Salzsäure wird mit 150 Gew.-Teilen konzentrierter wäßriger Natronlauge (ca. 60 Gew.-%) bei 20 bis 30 °C Innentemperatur neutralisiert und die Lösung auf einen pH von 8,5 gegracht. Die Lösung wird viermal mit 500 Volumenteilen n-Butanol extrahiert, der Butanolextrakt wird bei einer Badtemperatur von 50 bis 60 °C unter vermindertem Druck auf 750 Volumenteile eingeengt und gekühlt. Ausfallendes Kochsalz wird abfiltriert und in das Filtrat werden bei 20 bis 30 °C 40 Gew.-Teile gasförmiger Chlorwasserstoff eingeleitet. Die Lösung wird auf 10 °C abgekühlt, und die ausfallenden Kristalle werden abfiltriert und mit wenig Aceton gewaschen. Man erhält 95 Gew.-Teile 4-Methyl-5-hydroxymethylimidazol-hydrochlorid. Durch Einengen der Mutterlauge werden weitere 14 Gew.-Teile erhalten.

Der Rückstand aus der Mutterlauge ergibt nach zweimaliger fraktionierter Kristallisation mit 1-Propanol nochmals 13 und 2 Gew.-Teile Kristallisat, das identisch mit dem Erstkristallisat ist. Die Rohausbeute an 4-Methyl-5-hydroxymethylimidazol-hydrochlorid beträgt 124 Gew.-Teile (83,5 % d.Th.) vom Schmelzpunkt 222 bis 229 °C.

Die Verbindung ist nach HPLC und NMR einheitlich.

Beispiel 6

Man verfährt wie bei Beispiel 1, jedoch werden 82 Gew.-Teile 4-Methylimidazol (93,3 %ig) eingesetzt. Die wäßrig salzsaure Lösung wird unter vermindertem Druck bei 35 bis 40 °C Innentemperatur eingeengt zu einem kristallinen Rückstand von 168 Gew.-Teilen.

Variante A

84 Gew.-Teile des Kristallisats werden mit 350 Vol.-Teilen Butanol umkristallisiert. Nach dem Absaugen und Trocknen werden 41 Gew.-Teile 4-Methyl-5-hydroxymethylimidazol-hydrochlorid (59 % d.Th.) mit einem Schmelzpunkt von 240 bis 245 °C erhalten.

Variante B

84 Gew.-Teile des Kristallisats werden mit 84 Vol.-Teilen n-Butanol bei Raumtemperatur gerührt. Das Butanol wird abfiltriert. Es werden 48,6 Gew.-Teile (70 % d.Th.) 4-Methyl-5-hydroxymethylimidazol-hydrochlorid vom Schmelzpunkt 240 bis 245 °C erhalten.

Beispiel 7

110 Gew.-Teile 2-Ethyl-4-methylimidazol werden mit 560 Vol.-Teilen Wasser, 450 Gew.-Teilen konzentrierter Salzsäure (ca. 36 %) und 100 Gew.-Teilen wäßriger Formaldehydlösung (30 Gew.-%) 48 Stunden bei ca. 1 bar und 120 °C unter Rückfluß erhitzt und unter vermindertem Druck eingeengt. Nach dem Umkristallisieren des Rückstandes aus Ethanol werden 144,32 Gew.-Teile (82 % d.Th.) 2-Ethyl-4-methyl-5-hydroxymethylimidazol-hydrochlorid vom Schmelzpunkt 237 bis 238 °C erhalten.

Beispiel 8

103 Gew.-Teile 4-Ethylimidazol (93,5 %ig), 560 Vol.-Teile Wasser, 450 Gew.-Teile wäßrige konzentrierte Salzsäure (ca. 36 %) und 100 Gew.-Teile wäßrige Formaldehydlösung (30 Gew.-%) werden 48 Stunden bei 120 °C unter Druck (ca. 1 bar) erhitzt und unter vermindertem Druck eingeengt.

Es werden 193 Gew.-Teile eines teilweise kristallinen Rückstandes erhalten. Zur Reinigung wird in heißem Isorpopanol gelöst und durch Versetzen mit Aceton bei Raumtemperatur das 4-Ethyl-5-hydroxymethylimidazol-hydrochlorid ausgefällt.

Es werden 130,4 Gew.-Teile (80 % d.Th.) vom Schmelzpunkt 135-137 °C erhalten.

**Ansprüche**

1. Verfahren zur Herstellung von 5-Hydroxymethylimidazolen der Formel 1

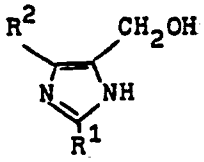

in der $R^1$ Wasserstoff, einen Alkylrest mit 1 bis 18 C-Atomen, Phenyl oder Aralkyl und $R^2$ einen Alkylrest mit 1 bis 18 C-Atomen oder Phenyl bedeuten, durch Hydroxymethylierung mit Formaldehyd, dadurch gekennzeichnet, daß man ein Imidazol der Formel 2

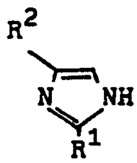

in der $R^1$ und $R^2$ die für Formel 1 angegebenen Bedeutungen hat, mit Formaldehyd oder einem Oligomeren des Formaldehyds in wäßriger salzsaurer Lösung mit einem Gehalt von 5 bis 18 Gew.-% Chlorwasserstoff, bezogen auf das vorhandene Wasser, bei Temperaturen von 80 bis 160 °C und gegebenenfalls in einem geschlossenen System unter Druck bei einem Molverhältnis von Imidazol zu Chlorwasserstoff von 1 : 1,5 bis 1 : 5 umsetzt und das erhaltene 5-Hydroxymethylimidazol in Form seines Hydrochlorids isoliert.

2. Verfahren nach Anspruch 1, dadurch gekennzeichnet, daß das Molverhältnis von Imidazol zu Formaldehyd 1 : 1 bis 1 : 1,5 beträgt.

3. Verfahren nach Anspruch 1 und 2, dadurch gekennzeichnet, daß man nach Beendigung der Reaktion in dem Reaktionsgemisch unter Verwendung einer Base einen pH-Wert von 7 bis 12 einstellt, anschließend das 5-Hydroxymethylimidazol mit einem mit Wasser nicht mischbaren organischen Lösungsmittel extrahiert und durch Einleiten von Chlorwasserstoff aus dem organischen Lösungsmittel als Hydrochlorid wieder fällt.

**Claims**

1. A process for the preparation of a 5-hydroxymethylimidazole of the formula 1

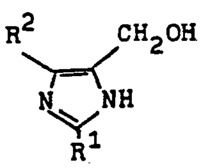

where $R^1$ is hydrogen, alkyl of 1 to 18 carbon atoms, phenyl or aralkyl, and $R^2$ is alkyl of 1 to 18 carbon atoms or phenyl, characterized in that an imidazole of the formula 2

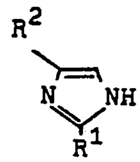

where $R^1$ and $R^2$ have the meanings given for formula 1, is reacted with formaldehyde or an oligomer of formaldehyde in aqueous hydrochloric acid solution, containing from 5 to 18 % by weight of hydrogen chloride, at from 80 to 160 °C, if appropriate in a closed system under pressure, the molar ratio of imidazole to hydrogen chloride being from 1 : 1.5 to 1 : 5, and the resulting 5-hydroxymethylimidazole is isolated in the form of its hydrochloride.

2. A process as claimed in claim 1, characterized in that the molar ratio of imidazole to formaldehyde is from 1 : 1 to 1 : 1.5.

3. A process as claimed in claims 1 and 2, characterized in that, after completion of the reaction, the pH of the reaction mixture is brought to 7-12 by means of a base, and the 5-hydroxymethylimidazole is then extracted with a water-immiscible organic solvent and is reprecipitated as the hydrochloride from the organic solvent by introducing hydrogen chloride.

**Revendications**

1. Procédé de préparation de 5-hydroxyméthylimidazoles de formule 1

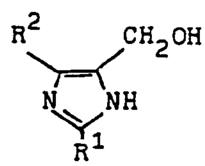

dans laquelle $R^1$ représente l'hydrogène, un reste alkyle à 1 à 18 atomes C, phényle ou aralkyle et $R^2$ un reste alkyle à 1 à 18 atomes C ou phényle, par hydroxyméthylation avec du formaldéhyde, caractérisé par le fait que l'on fait réagir un imidazole de formule 2

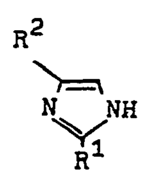

dans laquelle $R^1$ et $R^2$ ont les significations données pour la formule 1, avec du formaldéhyde ou un oligomère du formaldéhyde en solution chlorhydrique aqueuse d'une teneur de 5 à 18 % en poids d'acide chlohydrique, rapportée à l'eau

présente à des température de 80 à 160 °C et éventuellement, dans un système fermé, sous pression, avec un rapport molaire de l'imidazole à l'acide chlorhydrique de 1/1,5 à 1/5, et on isole le 5-hydroxyméthylimidazole obtenu, sous forme de chlorhydrate.

2. Procédé selon la revendication 1, caractérisé par le fait que le rapport molaire de l'imidazole à l'acide chlorhydrique est de 1/1 à 1/1,5.

3. Procédé selon la revendication 1 ou 2, caractérisé par le fait qu'après la fin de la réaction, on règle au pH de 7 à 12, dans le mélange réactionnel, en utilisant une base, puis on extrait le 5-hydroxyméthylimidazole avec un solvant organique, non miscible avec l'eau et on le refait précipiter du solvant organique, sous forme de chlorhydrate, par introduction d'acide chlorhydrique.